Europäisches Patentamt

European Patent Office

Office européen des brevets

(11) Veröffentlichungsnummer: **0 421 022 B1**

(12) **EUROPÄISCHE PATENTSCHRIFT**

(45) Veröffentlichungstag der Patentschrift: **04.08.93**

(51) Int. Cl.⁵: **A61K 37/54**

(21) Anmeldenummer: **89118626.4**

(22) Anmeldetag: **06.10.89**

(54) **Verwendung katabolischer Enzyme zur Bekämpfung einer HIV-Erst-Infektion.**

(43) Veröffentlichungstag der Anmeldung:
**10.04.91 Patentblatt 91/15**

(45) Bekanntmachung des Hinweises auf die
Patenterteilung:
**04.08.93 Patentblatt 93/31**

(84) Benannte Vertragsstaaten:
**AT BE CH DE ES FR GB GR IT LI LU NL SE**

(56) Entgegenhaltungen:
**EP-A- 0 309 602**

**ROTE LISTE, 1986, Nr. 85075, "Wobe-Mugos"**

**ROTE LISTE, 1986, Nr. 85074, "Wobe-Mugos"**

**ROTE LISTE, 1986, Nr. 41001, "Wobenzym"**

(73) Patentinhaber: **MUCOS EMULSIONSGESELL-
SCHAFT m.b.H.
Miraustrasse 17
W-1000 Berlin 27(DE)**

(72) Erfinder: **Ransberger, Karl
Gabriel-von-Seidl-Strasse 62
W-8022 Grünwald(DE)**
Erfinder: **Stauder, Gerhard, Dr.
Kathi-Kobus-Steig 1
W-8190 Wolfratshausen(DE)**

(74) Vertreter: **Patentanwälte Grünecker, Kinkeldey, Stockmair & Partner
Maximilianstrasse 58
W-8000 München 22 (DE)**

**Beschreibung**

Die vorliegende Erfindung betrifft die Verwendung der Enzyme Pankreatin, Bromelain, Papain, Lipase, Amylase, Trypsin und/oder Chymotrypsin für die Herstellung eines Arzneimittels zur Bekämpfung einer HIV-Erstinfektion.

Das Human-Immundeficience-Virus (HIV) gehört zu der Gruppe der Retroviren, d.h ihr RNA-Genom muß zunächst in DNA transkripiert werden, bevor diese DNA dann in die chromosomale DNA der Wirtszellen eintreten kann. HIV zeichnet sich wie zum Beispiel auch Grippeviren durch hohe Mutationsraten ihrer für die core-Proteine kodierenden Gene aus. Wegen der damit verbundenen Antigen-Variabilität ist eine stabile Immunisierung bisher nicht möglich.

Es sind bisher eine ganze Reihe von Agenzien mit anti-viralen Eigenschaften zur Bekämpfung von HIV mit wenig Erfolg untersucht worden. So ist das Immunstimulanz Interleukin-2 bisher ohne Erfolg bei Aids-Kranken eingesetzt worden. Bei Immunstimulanzien besteht dabei außerdem die Gefahr, daß sie durch eine mögliche Stimulierung von infizierten Zellen zur Verstärkung der Infektion beitragen können. Rückenmarktransplantationen und Leukozytentransfusionen haben bisher keinen Erfolg gehabt, da auch die neuen Zellen rasch infiziert wurden.

EP-A-0 309 602 offenbart die Verwendung katabolischer Enzyme zum Bekämpfen der erworbenen Immunschwäche (AIDS) und deren Vorstadien (LAS, ARC).

Die bisher beschriebenen Bekämpfungsmaßnahmen der Krankheit Aids richten sich gegen HIV-Sekundärinfektionen, d.h. die Bekämpfung der Krankheit in einem Stadium in dem bereits Immunkomplexe und Komplemente gebildet sind.

Um die Ausbreitung der Krankheit soweit wie möglich und solange wie möglich einzudämmen, bis eine Therapie zur Bekämpfung von Aids-Erkrankten entwickelt wird, ist es notwendig, die Vermehrung des Virus sofort nach einer möglichen Infektion zu bekämpfen.

Der vorliegenden Erfindung liegt daher die Aufgabe zugrunde, ein wirksames Mittel zur Bekämpfung einer HIV-Erstinfektion zur Verfügung zu stellen.

Diese Aufgabe wird erfindungsgemäß dadurch gelöst, daß mindestens eines der Enzyme Pankreatin, Bromelain, Papain, Lipase, Amylase, Trypsin und/oder Chymotrypsin für die Herstellung eines Mittels zur Bekämpfung einer HIV-Erstinfektion verwendet wird.

Es hat sich überraschenderweise gezeigt, daß die Enzyme Pankreatin, Bromelain, Papain, Lipase, Amylase, Trypsin und/oder Chymotrypsin die Replikation von Aids-Viren hemmen können.

Die erfindungsgemäß verwendeten Enzyme lassen sich aus den folgenden Rohmaterialien isolieren.

Pankreatin wird aus Schweine- oder Rinderpankreas gewonnen.

Bromelain ist ein proteolytisch wirksames Enyzm aus dem Preßsaft der Ananas.

Papain ist ein proteolytisches Enzym, das aus dem Milchsaft der unreifen, fleischigen Früchte des Melonenbaums Carica papaya gewonnen wird.

Lipasen gehören zu der Untergruppe der Esterasen und werden aus Pankreas oder dem Pilz Rhizopus arrhizus gewonnen.

Amylasen sind Glykosid-spaltende Enzyme, die beispielsweise aus Pankreas oder speziellen Mikroorganismen isoliert werden.

Trypsin und Chymotrypsin sind proteolytische Enzyme, die ebenfalls im Pankreas gebildet werden und in Verbindung mit anderen Enzymen bereits therapeutisch eingesetzt wurden.

Vorzugsweise wird als Lipase Triacylglycerollipase und/oder als Amylase $\alpha$-Amylase verwendet. Diese zeigen eine gute viruzide Wirkung an der Virus-Replikation.

Eine besondere Wirksamkeit zeigt sich bei Verwendung einer Kombination der Enzyme Pankreatin, Bromelain, Papain, Triacylglycerollipase, $\alpha$-Amylase, Trypsin und/oder Chymotrypsin. Neben der bemerkenswerten und unerwarteten Wirkung dieser Enzyme auf die Virus-Replikation hat die kombinierte Verwendung der genannten Enzyme weiterhin den Vorteil, daß auch bei einer Langzeitanwendung keine schädigenden Nebenwirkungen auftreten.

Weiterhin kann vorzugsweise zusätzlich Rutosid, ein zu den Flavonoiden gehörendes Glykosid, verwendet werden.

Eine besonders gute Wirksamkeit hat die kombinierte Verwendung von 50 - 200 mg, vorzugsweise 100 mg Pankreatin, 20 - 100 mg, vorzugsweise 45 mg Bromelain, 40 - 100 mg, vorzugsweise 60 mg Papain, 5 - 50 mg, vorzugsweise 10 mg Triacylglycerollipase, 5 - 50 mg, vorzugsweise 10 mg $\alpha$-Amylase, 10 - 30 mg, vorzugsweise 24 mg Trypsin, 1 - 10 mg, vorzugsweise 1 mg Chymotrypsin und 10 - 100 mg, vorzugsweise 50 mg Rutosid x $3H_2O$ pro Dosiseinheit.

Weiterhin können die zu verwendenden Präparate zusätzlich Serratia-Peptidase enthalten. Serratia-Peptidase kann aus einem Mikroorganismus der Gattung Serratia gewonnen werden.

Das zur Anwendung kommende Präparat kann weiterhin übliche Hilfs- und/oder Trägerstoffe enthalten. Die Beispiele erläutern die Erfindung.

Die Enzyme wurden zunächst in Toxizitätstests auf ihre Toxizität gegenüber humanen Lymphozyten untersucht. Ein Zehntel der jeweils halbmaximal toxischen Substanzkonzentration wurde anschließend für den HIV-Suppressionstest verwendet. Hierfür wurden humane Spenderlymphozyten mit einer Standard-Präparation von HIV infiziert. Die infizierten Zellen wurden in getrennten Ansätzen für 3 und 4 Tage in Gegenwart der zu untersuchenden Substanzen kultiviert. Jeweils zu Ende der Kulturdauer wurden die Kulturen auf Überstand und Zellen getrennt und in zellfreiem Überstand die Konzentration an freigesetztem, viralen core-Protein p24 gemessen. In den Zellen wurde die Konzentration an synthetisierter HIV-RNA bestimmt. Die folgende Tabelle zeigt die Hemmung der HIV-Replikation auf der Ebene des viralen Proteins p24 sowie der viralen RNA. Dabei wurde die jeweils nach 3 und 4 Tagen akkumulierten Konzentrationen an p24 bzw. RNA in Dreifachansätzen bestimmt: einerseits in den Kulturen, die die zu untersuchende Substanz in der angegebenen Konzentration enthielten, andererseits in Kulturen, die lediglich das Lösungsmittel in der gleichen Endkonzentration enthielten ("Kontrolle).

Um eine prozentuale Hemmwirkung zu ermitteln, wurden die jeweiligen Konzentrationen von p24 und virale RNA gegenüber der Kontrolle verrechnet und der antivirale Effekt auf einer Skala von 0 bis 9 bewertet. Dabei bedeutet "0" = 0 bis 10 % Hemmung, "1" bedeutet 10 bis 20 % Hemmung u.s.w.

## Tabelle 1

## Inhibierung der HIV-Replikation auf der RNA-Ebene

| | 3 Tage (pg RNA/ml) | %-Hemmung | antiviraler Effekt |
|---|---|---|---|
| Kontrolle | 86,5 | | |
| Trypsin | 1,1 | 98,8 | 9 |
| Bromelain | 0,6 | 99,4 | 9 |
| Chyomtrypsin | 0,0 | 100,0 | 9 |
| Pankreatin | 13,9 | 83,9 | 8 |
| Papain | 15,0 | 82,7 | 8 |

| | 4 Tage (pg RNA/ml) | %-Hemmung | antiviraler Effekt |
|---|---|---|---|
| Kontrolle | 462,4 | | |
| Trypsin | 35,4 | 92,4 | 9 |
| Bromelain | 17,0 | 96,3 | 9 |
| Chymotrypsin | 26,9 | 94,2 | 9 |
| Pankreatin | 352,2 | 23,8 | 2 |
| Papain | 232,8 | 49,7 | 4 |

Tabelle 2

Inhibierung der HIV-Replikation auf der Protein-Ebene

|  | 3 Tage (ng p24/ml) | %-Hemmung | antiviraler Effekt |
|---|---|---|---|
| Kontrolle | 27,2 | | |
| Trypsin | 1,6 | 94,0 | 9 |
| Bromelain | 8,6 | 67,2 | 6 |
| Chyomtrypsin | 8,1 | 70,1 | 7 |
| Pankreatin | 0,8 | 97,0 | 9 |
| Papain | 9,8 | 64,2 | 6 |

|  | 4 Tage (ng p24/ml) | %-Hemmung | antiviraler Effekt |
|---|---|---|---|
| Kontrolle | 163,7 | | |
| Trypsin | 60,9 | 62,8 | 6 |
| Bromelain | 0,8 | 99,5 | 9 |
| Chymotrypsin | 60,1 | 63,3 | 6 |
| Pankreatin | 89,4 | 45,4 | 4 |
| Papain | 102,4 | 37,5 | 3 |

Die Hemmung der HIV-Replikation bezieht sich jeweils auf eine Enzymkonzentration von 50 $\mu$g/ml.

**Patentansprüche**

1. Verwendung mindestens eines der Enzyme Pankreatin, Bromelain, Papain, Lipase, Amylase, Trypsin und/oder Chymotrypsin zur Herstellung eines Arzneimittels zur Bekämpfung einer HIV-Erstinfektion.

2. Verwendung nach Anspruch 1,
   **dadurch gekennzeichnet,**
   daß als Lipase Triacylglycerollipase verwendet wird.

3. Verwendung nach Anspruch 1,
   **dadurch gekennzeichnet,**
   daß als Amylase $\alpha$-Amylase verwendet wird.

4. Verwendung nach Anspruch 1,
   **dadurch gekennzeichnet,**
   daß eine Kombination der Enzyme Pankreatin, Bromelain, Papain, Triacylglycerollipase, $\alpha$-Amylase, Trypsin und/oder Chymotrypsin verwendet wird.

**5.** Verwendung nach mindestens einem der Ansprüche 1-4,
**dadurch gekennzeichnet,**
daß zusätzlich Rutosid verwendet wird.

**6.** Verwendung nach Anspruch 5,
**dadurch gekennzeichnet,**
daß 50 - 200 mg, vorzugsweise 100 mg Pankreatin, 20 - 100 mg, vorzugsweise 45 mg Bromelain, 40 - 100 mg, vorzugsweise 60 mg Papain, 5 - 50 mg, vorzugsweise 10 mg Triacylglycerollipase, 5 - 50 mg, vorzugsweise 10 mg $\alpha$-Amylase, 10 - 30 mg, vorzugsweise 24 mg Trypsin, 1 - 10 mg, vorzugsweise 1 mg Chymotrypsin und 10 - 100 mg, vorzugsweise 50 mg Rutosid x $3H_2O$ pro Dosiseinheit verwendet werden.

**7.** Verwendung nach mindestens einem der Ansprüche 1-6,
**dadurch gekennzeichnet,**
daß zusätzlich Serratia-Peptidase verwendet wird.

**Claims**

**1.** Use of at least one of the enzymes pancreatin, bromelain, papain, lipase, amylase, trypsin and/or chymotrypsin for the preparation of a pharmaceutical for controlling a first infection with HIV.

**2.** Use according to Claim 1, characterised in that triacylglycerol lipase is used as lipase.

**3.** Use according to Claim 1, characterised in that $\alpha$-amylase is used as amylase.

**4.** Use according to Claim 1, characterised in that a combination of the enzymes pancreatin, bromelain, papain, triacylglycerol lipase, $\alpha$-amylase, trypsin and/or chymotrypsin is used.

**5.** Use according to at least one of Claim 1-4, characterised in that rutoside is additionally used.

**6.** Use according to Claim 5, characterised in that 50-200 mg, preferably 100 mg, of pancreatin, 20-100 mg, preferably 45 mg, of bromelain, 40-100 mg, preferably 60 mg, of papain, 5-50 mg, preferably 10 mg, of triacylglycerol lipase, 5-50 mg, preferably 10 mg, of $\alpha$-amylase, 10-30 mg, preferably 24 mg, of trypsin, 1-10 mg, preferably 1 mg, of chymotrypsin and 10-100 mg, preferably 50 mg, of rutoside $\times$ $3H_2O$ are used per dose unit.

**7.** Use according to at least one of Claims 1-6, characterised in that serratia peptidase is additionally used.

**Revendications**

**1.** Application d'au moins une des enzymes pancréatine, bromélaïne, papaïne, lipase, amylase, trypsine et/ou chymotrypsine à la préparation d'un médicament pour combattre une primo-infection par HIV.

**2.** Application selon la revendication 1, caractérisée en ce qu'on utilise comme lipase la triacylglycérol lipase.

**3.** Application selon la revendication 1, caractérisée en ce qu'on utilise comme amylase l'$\alpha$-amylase.

**4.** Application selon la revendication 1, caractérisée en ce qu'on utilise une combinaison des enzymes pancréatine, bromélaïne, papaïne, triacylglycérol lipase, $\alpha$-amylase, trypsine et/ou chymotrypsine.

**5.** Application selon au moins une des revendications 1-4, caractérisée en ce qu'on utilise en outre le rutoside.

**6.** Application selon la revendication 5, caractérisée en ce qu'on utilise 50-200 mg, de préférence 100 mg de pancréatine, 20-100 mg, de préférence 45 mg de bromélaïne, 40-100 mg, de préférence 60 mg de papaïne, 5-50 mg, de préférence 10 mg de triacylglycérol lipase, 5-50 mg, de préférence 10 mg d'$\alpha$-

amylase, 10-30 mg, de préférence 24 mg de trypsine, 1-10 mg, de préférence 1 mg de chymotrypsine et 10-100 mg, de préférence 50 mg de rutoside x 3 $H_2O$ par unité posologique.

7.  Application selon au moins une des revendications 1-6, caractérisée en ce qu'on utilise en outre la serratia peptidase.